(19) Europäisches Patentamt
European Patent Office
Office européen des brevets

(11) Veröffentlichungsnummer: **0 003 114**
**B1**

(12) **EUROPÄISCHE PATENTSCHRIFT**

(45) Veröffentlichungstag der Patentschrift: **27.01.82**

(51) Int. Cl.³: **C 07 D 213/64,** A 01 N 43/40

(21) Anmeldenummer: **79100035.9**

(22) Anmeldetag: **08.01.79**

(54) **Herbizid wirksame ungesättigte Ester von 4-(3',5'-Dihalogenpyridyl -(2')-oxy)-alpha-phenoxypropionsäuren, Verfahren zu ihrer Herstellung sowie sie enthaltende herbizide Mittel und deren Verwendung.**

(30) Priorität: **18.01.78 CH 513/78**

(43) Veröffentlichungstag der Anmeldung: **25.07.79 Patentblatt 79/15**

(45) Bekanntmachung des Hinweises auf die Patenterteilung: **27.01.82 Patentblatt 82/4**

(84) Benannte Vertragsstaaten: **BE CH DE FR GB IT NL**

(56) Entgegenhaltungen:
**DE-A-2 611 695**
**DE-A-2 623 558**
**FR-A-2 288 089**
**FR-A-2 359 129**

(73) Patentinhaber: **CIBA-GEIGY AG, Patentabteilung Postfach, CH-4002 Basel (CH)**

(72) Erfinder: **Böhner, Beat, Dr., Hügelweg 3, CH-4102 Binningen (CH)**
Erfinder: **Rempfler, Hermann, Dr., Allschwilerweg 67, CH-4102 Binningen (CH)**
Erfinder: **Schurter, Rolf, Dr., Holzmattstrasse 45, CH-4102 Binningen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 003 114 B1

# Herbizid wirksame ungesättigte Ester von 4-[3',5'-Dihalogenpyridyl-(2')-oxy]-α-phenoxypropionsäuren, Verfahren zu ihrer Herstellung sowie sie enthaltende herbizide Mittel und deren Verwendung

Vorliegende Erfindung betrifft neue herbizid wirksame ungesättigte Ester von 4-(3',5'-Dihalogenpyridyl-(2')-oxy)-α-phenoxy-propion- und propionthiolsäuren, Verfahren zu ihrer Herstellung, ferner herbizide Mittel, die diese neuen Verbindungen als Wirkstoffe enthalten, sowie die Verwendung der neuen Wirkstoffe und der sie enthaltenden Mittel zur selektiven Bekämpfung von Unkräutern in Kulturpflanzenbeständen.

In den letzten Jahren sind zahlreiche Derivate von para-substituierten Hydroxy-diphenyläthern bekannt geworden, vgl. DE-A-223 894, DE-A-2 433 067, DE-A-2 531 643, DE-A-2 649 706, DE-A-2 609 461, DE-A-2 611 695, DE-A-2 623 558, DE-A-2 628 384, DE-A-2 652 384, DE-A-2 730 591, DE-A-2 809 541.

Außerdem sind heterocyclische Analoga, wie beispielsweise entsprechend substituierte Pyridyl-phenyläther bekannt geworden, vgl. DE-A-2 546 251 bzw. FR-A 2 288 089, JP-A-1 139 627 und DE-A-2 732 846 bzw. FR-A 2 359 129.

Es wurde nun überraschenderweise gefunden, daß die im Pyridylring dihalogenierten neuen Wirkstoffe gemäß vorliegender Erfindung den in den Handel eingeführten Produkten und den strukturell nächst verwandten Verbindungen der FR-A 2 288 089 sowie den unter den Umfang der FR-A 2 359 129 fallenden nächstvergleichbaren trihalogenierten Analogen in der selektiven Unkrautbekämpfung klar überlegen sind.

Die neuen ungesättigten 4-(3',5'-Dihalogen-pyridyl-(2')-oxy)-α-phenoxy-propion- und propionthiolsäure-Ester vorliegender Erfindung entsprechen der Formel I

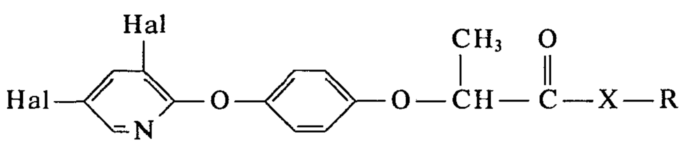

(I)

worin Hal unabhängig voneinander Chlor oder Brom, X ein Sauerstoff- oder Schwefelatom, und R einen gegebenenfalls durch Chlor substituierten geradkettigen oder verzweigten $C_3-C_9$-Alkinylrest, der neben der Dreifachbindung noch eine Doppelbindung enthalten kann, den 2-Äthinyl-cyclohexyl-(1)-Rest, einen geradkettigen oder verzweigten Cyanoalkylrest mit 1 bis 5 C-Atomen im Alkylteil, den 1-Cyano-cyclohexyl-(1)-Rest oder einen mit einem Sauerstoffatom X verbundenen Rest der Formel

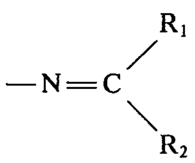

oder

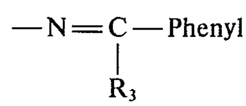

bedeutet, worin $R_1$ Wasserstoff oder einen Alkylrest mit 1 bis 4 C-Atomen und $R_2$ einen Alkylrest mit 1 bis 4 C-Atomen bedeuten, oder $R_1$ und $R_2$ zusammen mit dem benachbarten C-Atom einen Cyclopentan- oder Cyclohexanring bilden und $R_3$ Wasserstoff, Methyl oder eine Cyanogruppe bedeutet.

Alkinylreste R können geradkettig oder verzweigt sein, die Dreifachbindung kann endständig sein oder im Innern der $-C-C$-Kette liegen, es kann neben der Dreifachbindung noch eine Doppelbindung vorhanden sein. Die Alkinylreste können auch durch Chlor substituiert sein.

In der Formel I ist Hal vorzugsweise Chlor. Von den Resten R sind solche mit $-C{\equiv}C$-Dreifachbindung den Cyanalkyl- und den Oximestern wirkungsmäßig eher überlegen.

Die erfindungsgemäßen Wirkstoffe der Formel I und die sie als aktive Komponente enthaltenden herbiziden Mittel sind insbesondere brauchbar zur selektiven Bekämpfung von schwer bekämpfbaren Ungräsern in Kulturpflanzenbeständen, sowohl in dicotylen (Baumwolle, Soja) wie auch in monokotylen Kulturpflanzen, wie Reis, Weizen und anderen Getreidesorten. Die erfindungsgemäßen Verbindungen sind also gegenüber Kulturpflanzen gut verträglich und gegen Ungräser sehr wirksam.

Die bereits bekannten 2,4-Dichlorphenoxyphenoxyderivate des Standes der Technik wie die erwähnten gesättigten und die doppelt oder dreifach ungesättigten Verbindungen der Deutschen Offenlegungsschriften 2 223 894, 2 623 558, 2 628 384 und 2 611 695 befriedigen bezüglich Aktivität gegen schwierig zu bekämpfende Ungräser — insbesondere in tiefen Aufwandmengen — nicht vollständig.

Die bereits bekannten substituierten Pyridyloxyphenoxy-alkancarbonsäurederivate des Standes der Technik, wie die Ester und Thioester der FR-A 2 288 089 sowie Nitrile und Ester mit anderen Substituenten im Pyridyloxyrest sind gegenüber empfindlichen Kulturpflanzen wie z. B. Weizen entweder zu aggressiv oder zeigen bei guter Verträglichkeit eine zu geringe Aktivität gegenüber den zu bekämpfenden Ungräsern. Die vergleichbaren ungesättigen Ester von im Pyridylring dreifach

substituierten Pyridyloxyphenoxy-propionsäuren der FR-A 2 359 129 sind herbizid nur schwach wirksam.

Überraschenderweise zeichnen sich die erfindungsgemäßen Wirkstoffe gegenüber den bekannten gesättigten Estern und Thiolestern der Pyridyloxy-$\alpha$-phenoxy-propionsäurereihe durch eine deutlich besser Verträglichkeit gegenüber Kulturpflanzen, wie insbesondere Weizen, Gerste und Reis aus (Selektivität), gegenüber bekannten aromatisch ungesättigten Estern und Thiolestern durch eine bessere Aktivität gegen Ungräser, wie insbesondere Avena fatua (Wildhaferarten).

Aufgabe dieser Erfindung war also, neue Ester in der Reihe der halogenierten 4-(Pyridyloxy)-$\alpha$-phenoxypropion- und -propionthiolsäuren zu schaffen, welche bekannten Verbindungen ähnlicher Struktur in der herbiziden Wirkung gegen schwer bekämpfbare Ungräser überlegen und gegenüber wichtigen Kulturpflanzen wie Weizen, Gerste und Reis verträglicher sind, also eine Bereicherung der Technik darstellen.

Zur Herstellung der neuen Ester der Formel I dienen an sich bekannte Verfahren:

Nach einem dieser Verfahren setzt man ein entsprechendes 4-(3′,5′-Dihalogen-pyridyl-(2′)-oxy)-$\alpha$-phenoxy-propionsäurehalogenid der Formel II

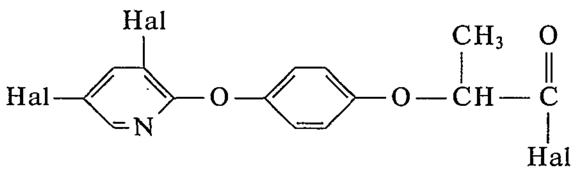

(II)

worin die »Hal« Halogenatome darstellen, in Gegenwart eines basischen Säureakzeptors, mit einem Alkohol oder Thiol der Formel III

$$H—X—R \qquad (III)$$

um, worin R und X wie unter Formel I definiert sind.

Gemäß einem weiteren Verfahren setzt man den entsprechenden Hydroxy-phenyl-pyridyläther oder ein Salz desselben, von der Formel IV

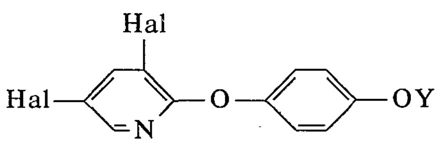

(IV)

worin Y Wasserstoff oder das Äquivalent eines Alkalimetall- oder Erdalkalimetallkations bedeutet, mit einem $\alpha$-Halogenpropion(thiol)säureester der Formel V

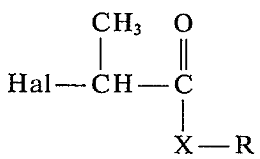

(V)

in Gegenwart eines säurebindenden Mittels (Base) um.

Die Umsetzungen werden vorzugsweise in einem gegenüber den Reaktionskomponenten inerten Lösungsmittel durchgeführt. Als Lösungsmittel kommen solche aus den verschiedensten Stoffklassen in Frage, wie aliphatische und aromatische, gegebenenfalls chlorierte Kohlenwasserstoffe, z. B. Äthylenchlorid etc., sowie polare organische Lösungsmittel, wie Alkohole, Äther, Ketone, Amide, stabile Ester, z. B. Methyläthylketon, Dimethoxyäthan, Dimethylformamid, Dimethylsulfoxid, Tetrahydrofuran.

Als basische Säureakzeptoren für die Umsetzung mit den Halogenverbindungen der Formel II und V können wässerige Alkalimetallhydroxide, wie KOH und NaOH sowie weitere übliche basische Stoffe, wie Karbonate ($K_2CO_3$, $NaHCO_3$), Alkoholate ($NaOCH_3$ und Kalium-tert.-butylat), aber auch organische Basen wie Triäthylamin verwendet werden.

Die Ausgangsstoffe der Formeln II bis V sind zum Teil bekannt oder können nach bekannten Verfahren hergestellt werden.

Zu den Propion- und Propionthiol-säureestern der Formel I (X = O oder S) kann man auch gelangen, indem man die entsprechende freie 4-(3′,5′-Dihalogen-pyridyl-(2′)-oxy)-$\alpha$-phenoxy-propion(thiol)säure bzw. ein Metallsalz dieser Säure mit einem ungesättigten Halogenid der Formel Hal—R in Gegenwart einer Base umsetzt.

Die Herstellung der dazu verwendbaren freien Propionthiolsäure und ihrer Metallsalze erfolgt aus

dem entsprechenden Propionsäurehalogenid mit Schwefelwasserstoff, $Na_2S$ oder NaHS in Gegenwart eines basischen Säureakzeptors. Die nach dieser Methode hergestellte 4-(3',5'-Dichlor-pyridyl-(2')oxy)-α-phenoxy-propionthiolsäure ist ein Öl vom Brechungsindex $n_D^{21} = 1{,}5787$, das nach Kristallisation bei 85–87° C schmilzt.

Schließlich kann man zur Herstellung von ungesättigten Estern der 4-(3',5'-Dihalogenpyridyl-(2')-oxy-α-phenoxy-propionsäure (X = Sauerstoff) die freie Säure auch direkt mit einem entsprechenden Alkohol nach bekannten Methoden verestern.

Die nachfolgenden Beispiele veranschaulichen die Herstellung einiger erfindungsgemäßer Wirkstoffe der Formel I. Weitere in entsprechender Weise oder nach einer anderen der im Text erwähnten Methoden hergestellte Endstoffe der Formel I sind anschließend tabellarisch aufgeführt.

## Beispiel 1

10,2 g (0,04 Mol) 4-(3',5'-Dichlorpyridyl-2'-oxy)-phenol, 8,4 g (0,044 Mol) α-Brompropionsäurepropargylester und 8,5 g (0,06 Mol) Kaliumcarbonat werden in 120 ml Äthylmethylketon 6 Std. unter Rückfluß erhitzt. Die Salze werden abfiltriert, das Filtrat eingedampft. Zur Reinigung wird das Produkt in Chloroform über eine kurze Kieselgelsäule filtriert. Nach dem Abdampfen des Chloroforms erhält man 9 g (62%) α-[4-(3',5'-Dichlorpyridyl-2'-oxy)-phenoxy]-propionsäure-propargylester als hellgelbes Öl mit einem Brechungsindex von $n_D^{40} = 1{,}5524$. (Verbindung Nr. 1)

## Beispiel 2

26,8 g (0,082 Mol α-(4-(3',5'-Dichlorpyridyl-2'-oxy)-phenoxy)-propionsäure werden mit 30 ml Thionylchlorid versetzt und, nachdem die Gasentwicklung abgeklungen ist, auf 50° C erwärmt. Nach 2 Std. wird das Reaktionsgemisch am Vacuum eingedampft, mit 100 ml Toluol versetzt und wieder eingedampft. Als Produkt erhält man ein dunkelbraunes Öl, das langsam zu kristallisieren beginnt. Es werden 25,9 g (86,7%) α-(4-(3',5'-Dichlorpyridyl-2'-oxy)-phenoxy)-propionsäurechlorid vom Schmelzpunkt 45° C erhalten.

17,3 g (0,05 Mol) dieses Säurechlorids werden zu einem Gemisch von 4,6 g (0,055 Mol) 70%igem, wässerigem Glycolsäurenitril, 7,6 g (0,055 Mol) Triäthylamin und 100 ml Methylenchlorid getropft, wobei die Temperatur auf 35° C ansteigt. Nach einer Stunde versetzt man das Reaktionsgemisch mit 100 ml Wasser. Die organische Phase filtriert man über eine kleine Kieselgelsäule und erhält beim Eindampfen des Filtrats 14,7 g (80,3%) α-(4-(3',5'-Dichlorpyridyl-2'-oxy)-phenoxy)-propionsäure-cyanomethylester in Form weißer Kristalle vom Schmelzpunkt 67–68° C (Verbindung Nr. 20).

## Beispiel 3

### Herstellung der freien Propionthiolsäure als Ausgangsstoff

17,2 g (0,0496 Mol) α-(4-(3',5'-Dichlorpyridyl-2'-oxy)-phenoxy)-propionsäurechlorid, hergestellt gemäß Beispiel 2, werden einem Gemisch von 8,9 g KOH in 4,9 ml Wasser und 75 ml Dimethoxyäthan zugetropft, das bei 10–15° C unter starkem Rühren mit $H_2S$ gesättigt wurde. Während des Zutropfens hält man die Temperatur mit einem Eisbad bei 10° C. Anschließend läßt man 30 Min. bei Raumtemperatur ausrühren und gießt das Reaktionsgemisch auf 150 ml Eis/Wasser. Die trübe, braune Lösung wird mit conc. HCl auf pH 1 gestellt. Dabei fällt ein braunes Öl aus, das in Methylenchlorid aufgenommen wird. Die organische Phase wird direkt auf eine kleine Kieselgelsäule gegeben und mit Methylenchlorid durchgespült. Nach dem Eindampfen der hellgelben Lösung erhält man ein oranges, klares Öl. Dieses kristallisiert beim Verreiben mit Petroläther. Man erhält 16,8 g α-(4-(3',5'-Dichlorpyridyl-2'-oxy)-phenoxy)-propionthiolsäure als gelbe Kristalle vom Schmelzpunkt 85–87° C.

## Beispiel 4

17,2 g (0,05 Mol) α-(4-(3',5'-Dichlorpyridyl-2'-oxy)-phenoxy)-propionsäurechlorid, hergestellt gemäß Beispiel 2, werden in 20 ml Methylenchlorid gelöst und bei 20° C zu dem vorgelegten Gemisch von 4,0 g (0,055 Mol) Acetonoxim, 7,3 ml NaOH 30%ig, 30 ml Wasser und 50 ml Methylenchlorid zugetropft. Anschließend läßt man Std. ausrühren und trennt die organische Phase ab. Diese gibt man direkt auf eine kleine Kieselgelsäule und spült das Produkt mit Methylenchlorid durch. Die farblose, klare Lösung dampft man am Wasserstrahlvacuum ein und erhält 8,4 g (44,0%) α-(4-(3',5'-Dichlorpyridyl-2'-oxy)-phenoxy)-propionsäure-aceton-oximester als klares, gelbliches Öl mit dem Brechungsindex von $n_D^{20} = 1{,}5678$ (Verbindung Nr. 36).

Beispiel 5

328 g (1 Mol) α-(4-(3',5'-Dichlorpyridyl-2'-oxy)-phenoxy)-propionsäure, 84 g (1,5 Mol) Propargylalkohol und 5 g p-Toluolsulfonsäure werden in Toluol 14 Std. an einem Wasserabscheider unter Rückfluß erhitzt. Die Lösung im Reaktionsgefäß wird eingedampft, in Äther aufgenommen und klarfiltriert. Die p-Toluolsulfonsäure wird durch Ausschütteln mit wässerigem Bicarbonat entfernt. Nach dem Trocknen mit Magnesiumsulfat wird der Äther eingedampft. Man erhält 355 g (96,9% d. Theorie) reinen Propargylester der Ausgangssäure als Öl, das langsam kristallisiert. Die Kristalle haben dann einen Schmelzpunkt von 62–65° C (vgl. auch Beispiel 1, Verbindung Nr. 1).

Die in den vorstehenden Beispielen beschriebenen sowie weitere in analoger Weise hergestellte Verbindungen der Formel I sind nachstehend tabellarisch angeführt.

1. 4 - (3',5' - Dichlorpyridyl - 2'-oxy) - α - phenoxy - propionsäure und -propionthiolsäure-ester der Formel

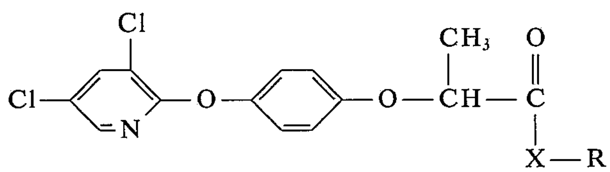

a) Alkinyl-Derivate

| Verbindung Nr. | X | R | Physikalische Daten $n_D$ | Smp. °C |
|---|---|---|---|---|
| 1 | O | $-CH_2-C\equiv CH$ | $n^{40} = 1{,}5524$ | 62–65° |
| 2 | S | $-CH_2-C\equiv CH$ | $n^{21} = 1{,}5988$ | 89–91° |
| 3 | S | $-CH_2-C\equiv C-CH_2-Cl$ | $n^{25} = 1{,}5945$ | |
| 4 | O | $-CH_2-C\equiv C-CH_2-Cl$ | $n^{25} = 1{,}5581$ | |
| 5 | O | 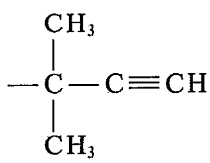 | $n^{25} = 1{,}5561$ | |
| 6 | O | 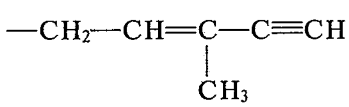 (trans) | $n^{25} = 1{,}5588$ | |
| 7 | O | 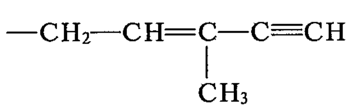 (cis) | $n^{25} = 1{,}5440$ | |
| 8 | O | 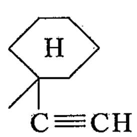 | | 90–92° |
| 9 | O | 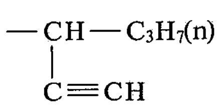 | $n^{30} = 1{,}5488$ | |

Fortsetzung

| Verbindung Nr. | X | R | Physikalische Daten $n_D$ | Smp. °C |
|---|---|---|---|---|
| 10 | O | 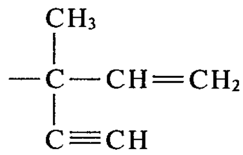 | $n^{30} = 1{,}5559$ | |
| 11 | O | 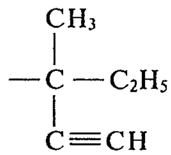 | $n^{30} = 1{,}5523$ | |
| 12 | O | 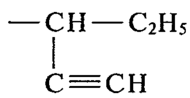 | | |
| 13 | O | 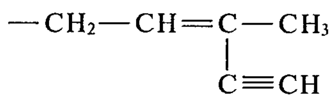 | | |
| 14 | O | 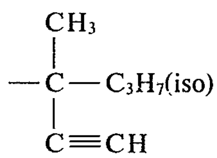 | | |
| 15 | O | 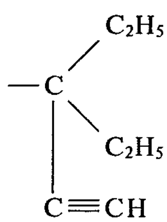 | | |
| 16 | O | 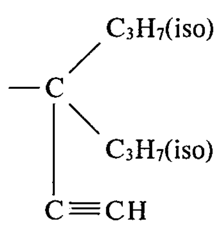 | | |
| 17 | O | 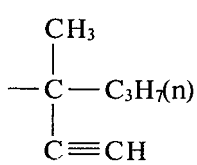 | | |

Fortsetzung

| Verbindung Nr. | X | R | Physikalische Daten $n_D$ | Smp. °C |
|---|---|---|---|---|
| 18 | O | 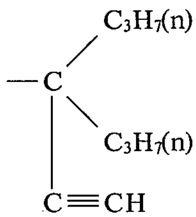 | | |
| 19 | O | 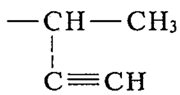 | | |

b) Cyanalkyl-Derivate

| Verbindung Nr. | X | R | Physikalische Daten $n_D$ | Smp. °C |
|---|---|---|---|---|
| 20 | O | $-CH_2-CN$ | | 67–68° |
| 21 | O | 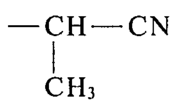 | $n^{20} = 1{,}5564$ | |
| 22 | S | $-CH_2-CN$ | | 85–88° |
| 23 | S | $-CH_2-CH_2-CN$ | $n^{20} = 1{,}5806$ | |
| 24 | S | $-CH_2-CH_2-CH_2-CN$ | $n^{20} = 1{,}5630$ | |
| 25 | O | $-CH_2-CH_2-CN$ | | 98–100° |
| 26 | S | 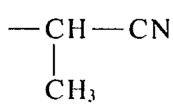 | $n^{25} = 1{,}5623$ | |
| 27 | O | 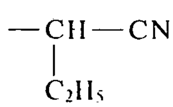 | $n^{25} = 1{,}5478$ | |
| 28 | O | 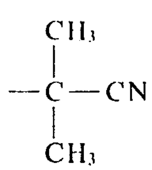 | | 73–75° |

Fortsetzung

| Verbindung Nr. | X | R | Physikalische Daten $n_D$ | Smp. °C |
|---|---|---|---|---|
| 29 | S | 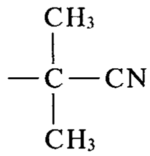 | | |
| 30 | O | 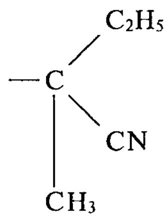 | | |
| 31 | O | 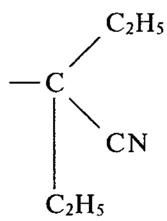 | | |
| 32 | S | 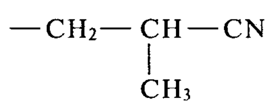 | | |
| 33 | O | 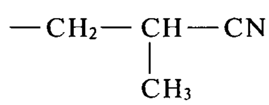 | | |
| 34 | S | 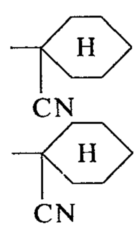 | | |
| 35 | O | | | |

c) Oxim-Derivate

| Verbindung Nr. | X | R | Bezeichnung bzw. physikalische Daten $n_D$ | Smp. °C |
|---|---|---|---|---|
| 36 | O | 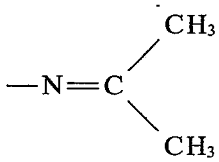 | $n^{20} = 1{,}5678$ | |
| 37 | O | 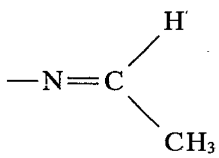 | Acetoximester | |
| 38 | O | 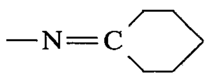 | $n^{25} = 1{,}5667$ | |
| 39 | O | 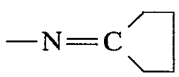 | Cyclopentanonoximester | |
| 40 | O | 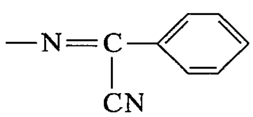 | $n^{25} = 1{,}5539$ | 96–99° |
| 41 | O | 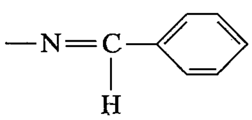 | Benzaldoximester | |
| 42 | O | 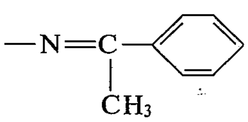 | Acetophenoximester | |

Weitere Ketoximester des Taps der Verbindung Nr. 36:

43) Methyl-isobutyl-ketoximester

44) Methyl-äthyl-ketoximester

45) Diäthyl-ketoximester

46) Methyl-propyl-ketoximester

47) Methyl-tert.butyl- ketoximester

2. 4-(3',5'-Dibrompyridyl-2'-oxy)-α-phenoxy-propionsäure und -propionthiolsäureester der Formel

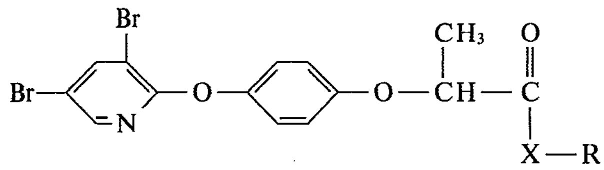

| Verbindung Nr. | X | R | Physikalische Daten $n_D$ | Smp. °C |
|---|---|---|---|---|
| 48 | S | $-CH_2-C\equiv CH$ | $n^{25} = 1{,}6120$ | |
| 49 | O | $-CH_2-C\equiv CH$ | $n^{40} = 1{,}5672$ | |
| 50 | O | 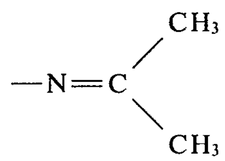 | | |
| 51 | O | 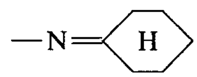 | | |
| 52 | O | $-CH_2-CN$ | | |
| 53 | O | 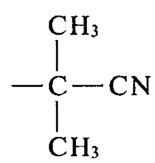 | | |

Die Erfindung betrifft auch herbizide Mittel, welche einen neuen Wirkstoff der Formel I enthalten, sowie Verfahren zur pre- und insbesondere post-emergenten Unkrautbekämpfung, insbesondere von monocotylen Ungräsern.

Die erfindungsgemäßen Mittel können in den üblichen Formulierungen vorliegen.

Die Herstellung erfindungsgemäßer Mittel erfolgt in an sich bekannter Weise durch inniges Vermischen und Vermahlen von Wirkstoffen der Formel I mit geeigneten Trägerstoffen, gegebenenfalls unter Zusatz von gegenüber den Wirkstoffen inerten Dispersions- oder Lösungsmitteln. Die Wirkstoffe können in den folgenden Aufarbeitungsformen vorliegen und angewendet werden:

Feste Aufarbeitungsformen:
  Stäubemittel, Streumittel, Granulate, Umhüllungsgranulate, Imprägnierungsgranulate und Homogengranulate;
in Wasser dispergierbare Wirkstoffkonzentrate:
  Spritzpulver (wettable powder), Pasten, Emulsionen;
flüssige Aufarbeitungsformen:
  Lösungen.

Zur Herstellung fester Aufarbeitungsformen (Stäubemittel, Streumittel, Granulate) werden die Wirkstoffe mit festen Trägerstoffen vermischt. Als Trägerstoffe kommen zum Beispiel Kaolin, Talkum, Bolus, Löß, Kreide, Kalkstein, Kalkgrits, Ataclay, Dolomit, Diatomeenerde, gefällte Kieselsäure, Erdalkalisilikate, Natrium- und Kaliumaluminiumsilikate (Feldspäte und Glimmer), Calcium- und Magnesiumsulfate, Magnesiumoxid, gemahlene Kunststoffe, Düngemittel, wie Ammoniumsulfat, Ammoniumphosphat, Ammoniumnitrat, Harnstoff, gemahlene pflanzliche Produkte, wie Getreidemehl, Baumrindemehl, Holzmehl, Nußschalenmehl, Cellulosepulver, Rückstände von Pflanzenextraktionen, Aktivkohle etc., je für sich oder als Mischungen untereinander in Frage.

Granulate lassen sich herstellen, indem man die Wirkstoffe in einem organischen Lösungsmittel löst und die so erhaltene Lösung auf ein granuliertes Mittel, z. B. Attapulgit, $SiO_2$, Granicalcium oder Bentonit, aufbringt und dann das organische Lösungsmittel wieder verdampft.

Polymergranulate können hergestellt werden, indem man z. B. ein fertiges, poröses Polymerengranulat, wie Harnstoff/Formaldehyd-Polymerisate, Polyacrylnitril und Polyester, mit bestimmter Oberfläche und günstigem, vorausbestimmtem Absorptions/Desorptions-Verhältnis mit den Wirkstoffen, z. B. in Form ihrer Lösungen (in einem niedrig siedenden Lösungsmittel), imprägniert und das Lösungsmittel entfernt. Derartige Polymerengranulate können in Form von Mikrogranulaten mit Schüttgewichten von vorzugsweise 300 g/Liter bis 600 g/Liter auch mit Hilfe von Zerstäubern aufgebracht werden. Das Zerstäuben kann über ausgedehnte Behandlungsflächen mit Hilfe von Flugzeugen durchgeführt werden.

Granulate sind auch durch Kompaktieren des Trägermaterials mit den Wirk- und Zusatzstoffen und anschließendes Zerkleinern erhältlich.

Diesen Mitteln können ferner den Wirkstoff stabilisierende Zusätze und/oder nichtionische, antionaktive und kationenaktive Stoffe zugegeben werden, die beispielsweise die Haftfestigkeit der Wirkstoffe auf Pflanzen und Pflanzenteilen verbessern (Haft- und Klebemittel) und/oder eine bessere Benetzbarkeit (Netzmittel) sowie Dispergierbarkeit (Dispergatoren) gewährleistet. Als Klebemittel kommen beispielsweise die folgenden in Frage: Olein-Kalk-Mischung, Cellulosederivate (Methylcellulose, Carboxymethylcellulose), Hydroxyäthylenglykoläther von Mono- und Dialkylphenolen mit 5 bis 15 Äthylenoxidresten pro Molekül und 8 bis 9 Kohlenstoffatomen im Alkylrest, Ligninsulfonsäure, deren Alkalimetall- und Erdalkalimetallsalze, Polyäthylenglykoläther (Carbowaxe), Fettalkoholpolyglykoläther mit 5 bis 20 Äthylenoxidresten pro Molekül und 8 bis 18 Kohlenstoffatomen im Fettalkoholteil, Kondensationsprodukte von Äthylenoxid, Propylenoxid, Polyvinylpyrrolidone, Polyvinylalkohole, Kondensationsprodukte von Harnstoff-Formaldehyd sowie Latex-Produkte.

In Wasser dispergierbare Wirkstoffkonzentrate, d. h. Spritzpulver (wettable powder), Pasten und Emulsionskonzentrate stellen Mittel dar, die mit Wasser auf jede gewünschte Konzentration verdünnt werden können. Sie bestehen aus Wirkstoff, Trägerstoff, gegebenenfalls den Wirkstoff stabilisierenden Zusätzen, oberflächenaktiven Substanzen und Antischaummitteln und gegebenenfalls Lösungsmitteln.

Die Spritzpulver (wettable powder) und Pasten werden erhalten, indem man die Wirkstoffe mit Dispergiermitteln und pulverförmigen Trägerstoffen in geeigneten Vorrichtungen bis zur Homogenität vermischt und vermahlt. Als Trägerstoffe kommen beispielsweise die vorstehend für die festen Aufarbeitungsformen erwähnten in Frage. In manchen Fällen ist es vorteilhaft, Mischungen verschiedener Trägerstoffe zu verwenden. Als Dispergatoren können beispielsweise verwendet werden: Kondensationsprodukte von sulfoniertem Naphthalin und sulfonierten Naphthalinderivaten mit Formaldehyd, Kondensationsprodukte des Naphthalins bzw. von Naphthalinsulfonsäuren mit Phenol und Formaldehyd sowie Alkalimetall-, Ammonium- und Erdalkalimetallsalze von Ligninsulfonsäure, weiter Alkylarylsulfonate, Alkali- und Erdalkalimetallsalze der Dibutylnaphthalinsulfonsäure, Fettalkoholsulfate, wie Salze sulfatierter Hexadecanole, Heptadecanole und Salze von sulfatiertem Fettalkoholpolyäthylenglykoläther, das Natriumsalz von Oleylmethyltaurid, ditertiäre Acetylenglykole, Dialkyldilaurylammoniumchlorid und fettsaure Alkali- und Erdalkalimetallsalze.

Als Antischaummittel kommen zum Beispiel Silicone in Frage.

Die Wirkstoffe werden mit den oben aufgeführten Zusätzen so vermischt, vermahlen, gesiebt und passiert, daß bei den Spritzpulvern der feste Anteil eine Korngröße von 0,02 bis 0,04 und bei den Pasten von 0,03 mm nicht überschreitet. Zur Herstellung von Emulsionskonzentraten und Pasten werden Dispergiermittel, wie sie in den vorangehenden Abschnitten aufgeführt wurden, organische Lösungsmittel und Wasser verwendet. Als Lösungsmittel kommen beispielsweise die folgenden in Frage: Alkohole, Xylole, Toluol, Dimethylsulfoxid, N,N-dialkylierte Amide und Trialkylamine. Die Lösungsmittel müssen praktisch geruchlos, nicht phytotoxisch, den Wirkstoffen gegenüber inert und dürfen nicht leicht brennbar sein.

Ferner können die erfindungsgemäßen Mittel in Form von Lösungen angewendet werden. Hierzu wird der Wirkstoff bzw. werden mehrere Wirkstoffe der Formel I in geeigneten organischen Lösungsmitteln, Lösungsmittelgemischen, Wasser oder Gemischen von organischen Lösungsmitteln mit Wasser gelöst. Als organische Lösungsmittel können aliphatische und aromatische Kohlenwasserstoffe, deren chlorierte Derivate, Alkylnaphthaline, allein oder als Mischung untereinander verwendet werden.

Der Gehalt an Wirkstoff in den oben beschriebenen Mitteln liegt zwischen 0,1 und 95%, bevorzugt zwischen 1 und 80%. Anwendungsformen können bis hinab zu 0,001% verdünnt werden. Die Aufwandmengen betragen in der Regel 0,1 bis 10 kg AS/ha, vorzugsweise 0,25 bis 5 kg AS/ha. Die Wirkstoffe der Formel I können beispielsweise wie folgt formuliert werden (Teile bedeuten Gewichtsteile):

Spritzpulver

Zur Herstellung eines a) 50%igen, b) 25%igen und c) 10%igen Spritzpulvers werden folgende Bestandteile verwendet:

a) 50 Teile 4-(3',5'-Dichlor-pyridyl-2'-oxy)-α-phenoxy-propionsäure-propargylester,
5 Teile Natriumdibutylnaphthylsulfonat,
3 Teile Naphthalinsulfonsäuren-Phenolsulfonsäuren-Formaldehyd-Kondensat 3 : 2 : 1,
20 Teile Kaolin,
22 Teile Champagne-Kreide;

b) 25 Teile des obigen Wirkstoffes,
5 Teile Oleylmethyltaurid-Natrium-Salz,
2,5 Teile Naphthalinsulfonsäuren-Formaldehyd-Kondensat,
0,5 Teile Carboxymethylcellulose,
5 Teile neutrales Kalium-Aluminium-Silikat,
62 Teile Kaolin;

c) 10 Teile des obigen Wirkstoffes,
3 Teile Gemisch der Natriumsalze von gesättigten Fettalkoholen,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschließend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden. Derartige Suspensionen werden zur Bekämpfung von Unkräutern und Ungräsern in Kulturpflanzungen im Nachauflaufverfahren verwendet.

Paste

Zur Herstellung einer 45%igen Paste werden folgende Stoffe verwendet:

45 Teile 4-(3',5'-Dichlorpyridyl-2'-oxy)-α-phenoxy)-propionthiolsäure-S-propargylester,
5 Teile Natriumaluminiumsilikat,
14 Teile Cetylpolyäthylenglykoläther mit 8 Mol Äthylenoxid,
1 Teil Oleylpolyäthylenglykoläther mit 5 Mol Äthylenoxid,
2 Teile Spindelöl,
23 Teile Wasser,
10 Teile Polyäthylenglykol.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25%igen Emulsionskonzentrats werden

25 Teile 4-(3',5'-Dichlorpyridyl-2'-oxy)-α-phenoxy-propionsäure-O-(1-äthyl-1-methyl-propargyl)-ester,
10 Teile Mischung von Nonylphenolpolyoxyäthylen und Calcium-dodecylbenzol-sulfonat,
10 Teile Cyclohexanon,
55 Teile Xylol

miteinander vermischt. Dieses Konzentrat kann mit Wasser zu Emulsionen auf zur Anwendung geeignete Konzentrationen verdünnt werden.

Anstatt des jeweiligen in den vorhergehenden Formulierungsbeispielen angegebenen Wirkstoffes kann auch eine andere der von der Formel I umfaßten Verbindungen verwendet werden.

Erfindungsgemäße Mittel, die als aktive Komponente mindestens eine Verbindung der Formel I enthalten, eignen sich besonders zur selektiven Bekämpfung schwerbekämpfbarer monocotyler Ungräser, wie Avena fatua (Wildhafer), Rotboellia, Digitaria, Setaria etc. in pre- und insbesondere post-emergenter Anwendung in Kulturpflanzenbeständen, wie z. B. Weizen, Gerste, Reis, aber auch Soja, Baumwolle, Zuckerrohr etc.

## Pre-emergente Herbizid-Wirkung
## (Keimhemmung)

Im Gewächshaus wird unmittelbar nach der Einsaat der Versuchspflanzen in Saatschalen die Erdoberfläche mit einer wässerigen Dispersion der Wirkstoffe, erhalten aus einem 25%igen Emulsionskonzentrat resp. aus einem 25%igen Spritzpulver mit Wirkstoffen, die wegen ungenügender Löslichkeit nicht als Emulsionskonzentrat hergestellt werden können, behandelt. Es wurden vier verschiedene Konzentrationsreihen angewendet, entsprechend 4, 2, 1 und 0,5 kg Wirksubstanz pro Hektar. Die Saatschalen werden im Gewächshaus bei 22–25°C und 50–70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben
2–3 = sehr starke Wirkung
4–6 = mittlere Wirkung
7–8 = geringe Wirkung
9 = keine Wirkung (wie unbehandelte Kontrolle)

Als Versuchspflanzen dienen:

| | |
|---|---|
| hordeum (Gerste) | setaria italica |
| triticum (Weizen) | echinochloa crus galli |
| zea (Mais) | beta vulgaris |
| sorghum hybr. (Hirse) | sida spinosa |
| oryza (Reis) | sesbania exaltata |
| glycine (Soja) | amaranthus retroflexus |
| gossypium (Baumwolle) | sinapis alba |
| avena fatua | ipomea purpurea |
| lolium perenne | galium aparine |
| alopecurus myosuroides | pastinaca sativa |
| bromus tectorum | rumex sp. |
| cyperus esculentus | chrysanthemum leucum. |
| rottboellia exaltata | abutilon sp. |
| digitaria sanguinalis | solanum nigrum |

## Post-emergente Herbizid-Wirkung
## (Kontaktherbizid)

Ein oder mehrere Unkräuter und Kulturpflanzen, sowohl monocotyle wie dicotyle, wurden nach dem Auflaufen (im 4- bis 6-Blattstadium) mit einer wäßrigen Wirkstoffdispersion in verschiedenen Dosierungen (0,125; 0,25; 0,5 und 1 kg Wirksubstanz pro Hektar) auf die Pflanzen gespritzt und diese bei 23–26°C und 45–60% rel. Luftfeuchtigkeit gehalten. 15 Tage nach Behandlung wird der Versuch ausgewertet und das Ergebnis wie im pre-emergent-Versuch nach derselben Notenskala bonitiert.

In diesen post-emergent-Versuchen (Kontaktherbizidwirkung) wurden als Vergleichsverbindungen von den folgenden vorbekannten, gesättigten und ungesättigten Hydroxy-diphenyläther-Derivaten jeweils die strukturell nächstverwandten Verbindungen in den Test einbezogen.

| Verbindung | Formel | Literatur |
|---|---|---|
| A | 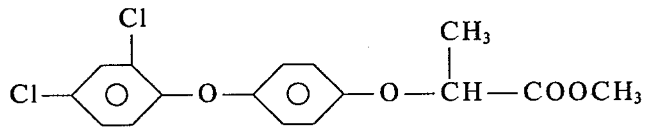 | DE-A-2 223 894 (Nr. 86) |
| B | 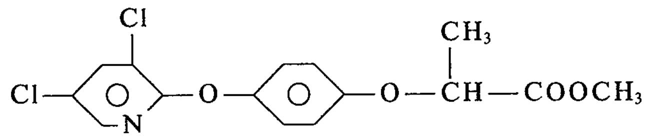 | DE-A-2 546 251 (Nr. 24) |
| C | 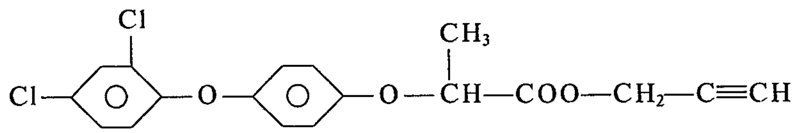 | DE-A-2 623 558 (Nr. 36) |
| D | 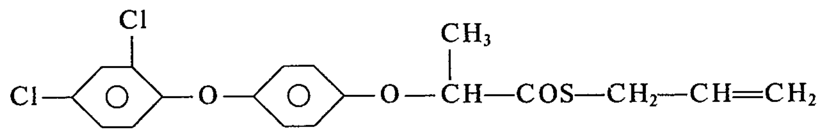 | DE-A-2 623 558 (Nr. 90) |
| E | 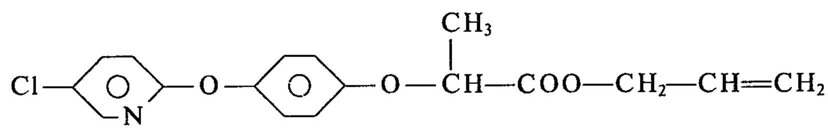 | DE-A-2 546 251 (Nr. 12) |
| F | 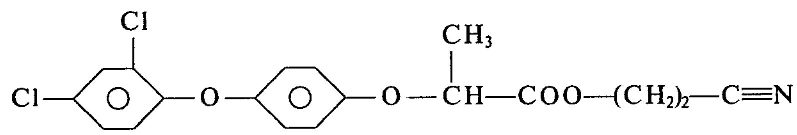 | DE-A-2 628 384 (Nr. 64) |
| G | 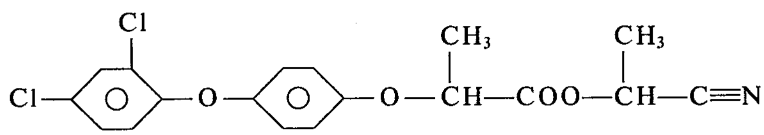 | DE-A-2 628 384 (Nr. 68) |
| H | 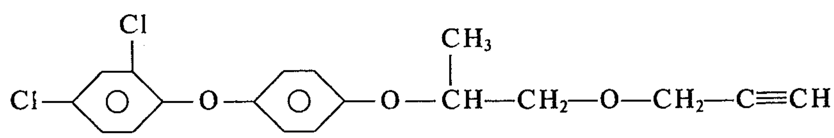 | DE-A-2 611 695 (Nr. 140) |
| I | 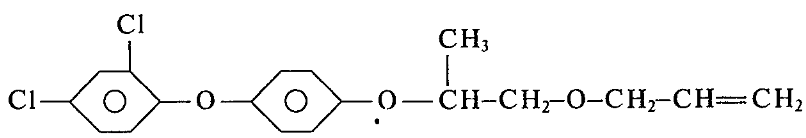 | DE-A-2 611 695 (Nr. 139) |
| K | 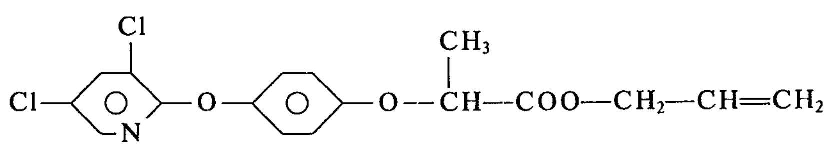 | DE-A-2 546 251 (Nr. 31) |

Erste Versuchsreihe

Kulturpflanze: Weizen (triticum) Sorte ›Probus‹

Unkraut: Wildhafer (Avena fatua)

Aufwandmengen: 1 kg und 0,5 kg Wirksubstanz pro Hektar

| Verbindung | Weizen | | Avena fatua | |
|---|---|---|---|---|
| No. | 1 kg | 0,5 kg | 1 kg | 0,5 kg |
| 1 | 4 | 9 | 1 | 1 |
| 2 | 2 | 8 | 1 | 1 |
| 3 | 9 | 9 | 1 | 2 |
| 4 | 9 | 9 | 1 | 1 |
| 5 | 5 | 9 | 1 | 1 |
| 6 | 5 | 9 | 1 | 1 |
| 7 | 6 | 7 | 1 | 1 |
| 8 | 9 | 9 | 1 | 2 |
| 9 | 4 | 9 | 1 | 1 |
| 10 | 9 | 9 | 1 | 1 |
| 11 | 9 | 9 | 1 | 1 |
| C | 9 | 9 | 4 | 7 |
| A | 9 | 9 | 3 | 5 |
| B | 2 | 3 | 1 | 1 |
| H | 9 | 9 | 9 | 9 |
| E | 4 | 5 | 2 | 4 |
| 20 | 2 | 5 | 1 | 1 |
| 21 | 3 | 9 | 1 | 1 |
| 22 | 4 | 9 | 1 | 1 |
| 23 | 7 | 9 | 1 | 2 |
| 24 | 7 | 9 | 1 | 2 |
| 25 | 9 | 9 | 1 | 4 |
| 26 | 8 | 9 | 1 | 1 |
| 27 | 3 | 9 | 1 | 1 |
| 36 | 6 | 9 | 1 | 1 |

Fortsetzung

| Verbindung | Weizen | | Avena fatua | |
|---|---|---|---|---|
| No. | 1 kg | 0,5 kg | 1 kg | 0,5 kg |
| 38 | 8 | 9 | 1 | 1 |
| 40 | 5 | 9 | 1 | 1 |
| 48 | 4 | 9 | 1 | 1 |
| 49 | 3 | 5 | 1 | 1 |
| F | 9 | 9 | 4 | 9 |
| G | 9 | 9 | 5 | 7 |

Zweite Versuchsreihe:

Kulturpflanze: Gerste (hordeum) Sorte ›Mazurka‹

Unkraut: Avena fatua (Wildhafer)

Aufwandmengen: 0,25 kg Wirksubstanz pro Hektar

| Verbindung Nr. | Gerste | Avena fatua |
|---|---|---|
| 4 | 7 | 3 |
| 8 | 9 | 2 |
| 21 | 7 | 3 |
| 23 | 9 | 3 |
| 24 | 7 | 3 |
| 26 | 7 | 2 |
| 36 | 9 | 2 |
| 40 | 8 | 3 |
| A | 9 | 7 |
| C | 9 | 8 |
| D | 9 | 9 |
| K | 3 | 2 |

Dritte Versuchsreihe

Kulturpflanze: Soja, Sorte ›Hark‹

Unkräuter: Hirsen (Rotboellia, Digitaria, Setaria)

Aufwandmengen: 0,5, 0,25 und 0,125 kg Wirksubstanz pro Hektar

| Verbindung | Soja | | | Rotboellia | | | Digitaria | | | Setaria | | |
|---|---|---|---|---|---|---|---|---|---|---|---|---|
| No. | 0,5 | 0,25 | 0,125 | 0,5 | 0,25 | 0,125 | 0,5 | 0,25 | 0,125 | 0,5 | 0,25 | 0,125 |
| 1 | 9 | 9 | 9 | 1 | 1 | 1 | 1 | 1 | 1 | 1 | 1 | 1 |
| 2 | 9 | 9 | 9 | 1 | 1 | 1 | 1 | 1 | 1 | 1 | 1 | 1 |
| 3 | 9 | 9 | 9 | 2 | 6 | 8 | 2 | 2 | 5 | 1 | 3 | 4 |
| 4 | 9 | 9 | 9 | 1 | 1 | 3 | 1 | 1 | 2 | 1 | 1 | 1 |
| 5 | 9 | 9 | 9 | 1 | 1 | 3 | 1 | 1 | 1 | 1 | 1 | 1 |
| 6 | 9 | 9 | 9 | 1 | 1 | 2 | 1 | 1 | 1 | 1 | 1 | 1 |
| 7 | 9 | 9 | 9 | 1 | 1 | 2 | 1 | 1 | 1 | 1 | 1 | 1 |
| 8 | 9 | 9 | 9 | 1 | 8 | 8 | 1 | 1 | 3 | 1 | 1 | 2 |
| 9 | 9 | 9 | 9 | 1 | 1 | 2 | 1 | 1 | 1 | 1 | 1 | 1 |
| 10 | 9 | 9 | 9 | 1 | 1 | 1 | 1 | 1 | 1 | 1 | 1 | 1 |
| 11 | 9 | 9 | 9 | 1 | 1 | 1 | 1 | 1 | 1 | 1 | 1 | 1 |
| C | 9 | 9 | 9 | 2 | 3 | 5 | 4 | 5 | 6 | 1 | 1 | 3 |
| H | 9 | 9 | 9 | 9 | 9 | 9 | 9 | 9 | 9 | 9 | 9 | 9 |
| I | 9 | 9 | 9 | 9 | 9 | 9 | 9 | 9 | 9 | 9 | 9 | 9 |
| 20 | 9 | 9 | 9 | 1 | 1 | 1 | 1 | 1 | 1 | 1 | 1 | 1 |
| 21 | 9 | 9 | 9 | 1 | 1 | 1 | 1 | 1 | 1 | 1 | 1 | 1 |
| 22 | 9 | 9 | 9 | 1 | 1 | 2 | 1 | 1 | 1 | 1 | 1 | 1 |
| 23 | 9 | 9 | 9 | 1 | 2 | 5 | 1 | 1 | 1 | 1 | 1 | 1 |
| 24 | 9 | 9 | 9 | 1 | 3 | 6 | 1 | 1 | 1 | 1 | 1 | 2 |
| 25 | 9 | 9 | 9 | 1 | 3 | 5 | 1 | 1 | 2 | 1 | 1 | 5 |
| 26 | 9 | 9 | 9 | 1 | 1 | 2 | 1 | 1 | 2 | 1 | 1 | 1 |
| 27 | 9 | 9 | 9 | 1 | 1 | 3 | 1 | 1 | 1 | 1 | 1 | 1 |
| 36 | 9 | 9 | 9 | 1 | 1 | 2 | 1 | 1 | 1 | 1 | 1 | 1 |
| 38 | 9 | 9 | 9 | 1 | 1 | 2 | 1 | 1 | 2 | 1 | 1 | 1 |

Fortsetzung

| Verbindung No. | Soja | | | Rotboellia | | | Digitaria | | | Setaria | | |
|---|---|---|---|---|---|---|---|---|---|---|---|---|
| | 0,5 | 0,25 | 0,125 | 0,5 | 0,25 | 0,125 | 0,5 | 0,25 | 0,125 | 0,5 | 0,25 | 0,125 |
| 40 | 9 | 9 | 9 | 1 | 1 | 3 | 1 | 1 | 2 | 1 | 1 | 1 |
| 48 | 9 | 9 | 9 | 1 | 2 | 6 | 1 | 2 | 2 | 1 | 1 | 2 |
| 49 | 9 | 9 | 9 | 1 | 1 | 2 | 1 | 1 | 2 | 1 | 1 | 2 |
| F | 9 | 9 | 9 | 5 | 7 | 9 | 6 | 7 | 8 | 2 | 4 | 7 |
| G | 9 | 9 | 9 | 4 | 7 | 9 | 4 | 7 | 8 | 2 | 3 | 7 |

Vierte Versuchsreihe

Kulturpflanze: Trockenreis, Sorte ›Caloro‹

Unkräuter: Digitaria, Setaria

Aufwandmengen: 0,5, 0,25 und 0,125 kg Wirksubstanz pro Hektar

| Verbindung No. | Trockenreis | | | Digitaria | | | Setaria | | |
|---|---|---|---|---|---|---|---|---|---|
| | 0,5 | 0,25 | 0,125 | 0,5 | 0,25 | 0,125 | 0,5 | 0,25 | 0,125 |
| 2 | 4 | 5 | 8 | 1 | 1 | 1 | 1 | 1 | 1 |
| 4 | 5 | 8 | 9 | 1 | 1 | 2 | 1 | 1 | 1 |
| 5 | 5 | 5 | 9 | 1 | 1 | 1 | 1 | 1 | 1 |
| 8 | 7 | 9 | 9 | 1 | 1 | 3 | 1 | 1 | 2 |
| 9 | 6 | 6 | 9 | 1 | 1 | 1 | 1 | 1 | 1 |
| 10 | 6 | 8 | 9 | 1 | 1 | 1 | 1 | 1 | 1 |
| 11 | 6 | 9 | 9 | 1 | 1 | 1 | 1 | 1 | 1 |
| C | 9 | 9 | 9 | 4 | 5 | 6 | 1 | 1 | 3 |
| H | 9 | 9 | 9 | 9 | 9 | 9 | 9 | 9 | 9 |
| I | 9 | 9 | 9 | 9 | 9 | 9 | 9 | 9 | 9 |
| 23 | 6 | 8 | 9 | 1 | 1 | 1 | 1 | 1 | 1 |
| 24 | 6 | 9 | 9 | 1 | 1 | 1 | 1 | 1 | 2 |
| 25 | 8 | 9 | 9 | 1 | 1 | 2 | 1 | 1 | 5 |
| 26 | 6 | 8 | 9 | 1 | 1 | 2 | 1 | 1 | 1 |
| 27 | 6 | 9 | 9 | 1 | 1 | 1 | 1 | 1 | 1 |
| 36 | 5 | 8 | 9 | 1 | 1 | 1 | 1 | 1 | 1 |
| 38 | 4 | 7 | 9 | 1 | 1 | 2 | 1 | 1 | 1 |
| 40 | 5 | 7 | 9 | 1 | 1 | 2 | 1 | 1 | 1 |
| 48 | 8 | 9 | 9 | 1 | 2 | 2 | 1 | 1 | 2 |
| 49 | 8 | 9 | 9 | 1 | 1 | 2 | 1 | 1 | 2 |
| F | 9 | 9 | 9 | 6 | 7 | 8 | 2 | 4 | 7 |
| G | 9 | 9 | 9 | 4 | 7 | 8 | 2 | 3 | 7 |

Es sind auch Feldversuche in den USA durchgeführt worden, wobei zwei erfindungsgemäße Verbindungen (Nr. 1 und 2) in Getreide bezüglich Phytotoxizität und Unkrautwirkung auf Avena fatua mit dem Handelsprodukt A und der Vergleichsverbindung B in ihrer Wirkung unter Anwendung verschiedener Dosen verglichen worden sind; die Aufwandmengen variierten zwischen 0,5 und 1,5 kg Wirksubstanz pro Hektar.

Sommergerste wurde 39 Tage nach der Aussaat, als sie sich im 4–6-Blattstadium befand und mit Avena fatua im 5–7-Blattstadium verunkrautet war, durch Spritzapplikation von oben mit den Wirkstoffdispersionen behandelt.

22 Tage nach dieser Behandlung zeigte Verbindung B bei der Aufwandmenge von 0,5 kg/ha schon eine 60%ige Schädigung der Gerste, während die erfindungsgemäßen Wirkstoffe 1 und 2 die Gerste nur zu 30% schädigten. Die Wirkung auf Avena fatua war für alle drei eingesetzten Wirkstoffe 100%ig.

Die erfindungsgemäßen Wirkstoffe zeichnen sich also durch eine bedeutend bessere Selektivität bei der zur völligen Vernichtung der Unkräuter ausreichenden Aufwandmenge von 0,5 kg/ha aus.

Winterweizen, der im Oktober gesät worden war, wurde im März, als er das 2–3-Blattstadium erreicht hatte und mit Avena fatua im 1–5-Blattstadium verunkrautet war, durch Spritzapplikation der wässerigen Wirkstoffpräparate von oben behandelt.

37 Tage nach der Behandlung zeigte Vergleichsverbindung B schon mit 0,5 kg/ha eine 50%ige Schädigung des Weizens, bei 0,75 l und 1,5 kg/ha eine 70%ige Schädigung des Weizens bei jeweils 100%iger Wirkung auf Avena fatua. Vergleichsverbindung A war weniger phytotoxisch (20%ige Weizenschädigung bei 0,5 bis 1,5 kg/ha), besaß aber eine nicht befriedigende Wirkung auf Avena fatua (nur 30% bei 0,5 kg/ha und 80% bei 1,5 kg/ha).

Die erfindungsgemäßen Verbindungen 1 und 2 zeigten ein bedeutend besseres Gesamtbild: Während Verbindung 1 bei voller Unkrautwirkung in allen Konzentrationen den Weizen zwischen 20 und 50% schädigte, zeigte Verbindung 2 bei 0,5 und 0,75 kg/ha überhaupt keine Schädigung des Weizens bei 90%iger Unkrautwirkung.

Ebenso gute Ergebnisse wurden mit Sommerweizen (Olaf) erzielt: 1 Monat nach der Saat erfolgte die post-emergente Spritzbehandlung, als der Weizen 3 Blätter und Avena fatua 2 bis 3 Blätter ausgebildet hatten.

15 Tage nach Applikation zeigte sich folgendes Bild:

Vergleichsverbindung b erzeugte bei 0,5, 0,75 und 1 kg/ha 30- bis 70%ige Schädigung des Weizens bei 60–90%iger Unkrautwirkung, Verbindung A ergab zwar in allen diesen Konzentrationsbereichen keine Schädigung des Weizens, aber nur eine 30- bis 50%ige Unkrautwirkung.

Demgegenüber zeigten die erfindungsgemäßen Wirkstoffe 1 und 2 bei den obigen Anwendungsmengen von 0,5, 0,75 und 1 kg/ha überhaupt keine Schädigung des Weizens bei 70- bis 90%iger Unkrautwirkung.

Daraus geht hervor, daß für jede der erfindungsgemäßen Verbindungen 1 und 2 in den erwähnten, mit Avena fatua verunkrauteten Getreidekulturen im Feldtest mindestens eine Aufwendungsdosis gewählt werden kann, die bei optimaler Unkrautwirkung eine minimale Schädigung des Getreides ergibt, was bei keiner der Vergleichsverbindungen der Fall ist: Diese sind bei jeder wählbaren Dosis entweder zu wenig unkrautwirksam oder zu phytotoxisch für das Getreide.

Ein Vergleichsversuch zwischen Verbindung 1 und dem vom FR-A 2 359 129 umfaßten, darin aber nicht genannten Propargylester der α-(4-(3′,5′,6′-Trichlorpyridyl-2′-oxy)-phenoxy)-propionsäure ergab eine ganz klare Überlegenheit von Verbindung 1, da die Herbizidwirkung des Propargylesters aus FR-A 2 359 129 völlig ungenügend war.

## Patentansprüche

1. Neue herbizid wirksame ungesättigte Ester von 4-(3′,5′-Dihalogenpyridyl-2′-oxy)-α-phenoxy-propion- und -propionthiolsäuren der Formel

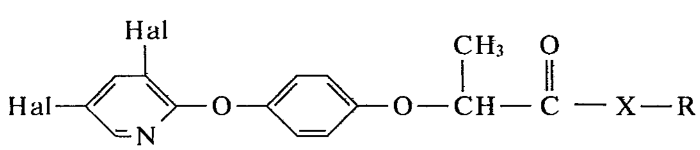

(I)

worin die Reste »Hal« unabhängig voneinander Chlor oder Brom, X ein Sauerstoff- oder Schwefelatom, R einen gegebenenfalls durch Chlor substituierten geradkettigen oder verzweigten $C_3$—$C_9$-Alkinylrest, der neben der Dreifachbindung noch eine Doppelbindung enthalten kann, den 2-Äthinyl-cyclohexyl-(1)-Rest, einen geradkettigen oder verzweigten Cyanoalkylrest mit 1 bis 5 C-Atomen im Alkylteil, den 1-Cyano-cyclohexyl-(1)-Rest oder einen mit einem Sauerstoffatom X verbundenen Rest der Formeln

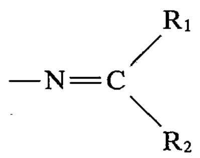

oder

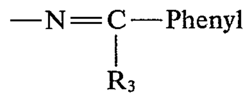

bedeutet, worin $R_1$ Wasserstoff oder einen Alkylrest mit 1 bis 4 C-Atomen und $R_2$ einen Alkylrest mit 1 bis 4 C-Atomen bedeuten, oder $R_1$ und $R_2$ zusammen mit dem benachbarten C-Atom einen Cyclopentan- oder Cyclohexanring bilden und $R_3$ Wasserstoff, Methyl oder eine Cyanogruppe bedeutet.

2. Ungesättigte Ester gemäß Patentanspruch 1, dadurch gekennzeichnet, daß »Hal« in der Formel I je Chlor bedeutet.

3. Die Verbindung der Formel

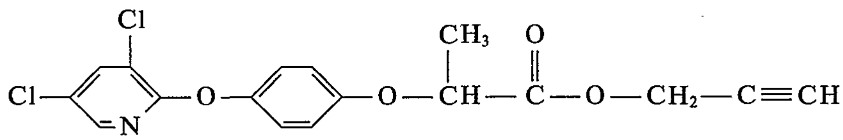

4. Die Verbindung der Formel

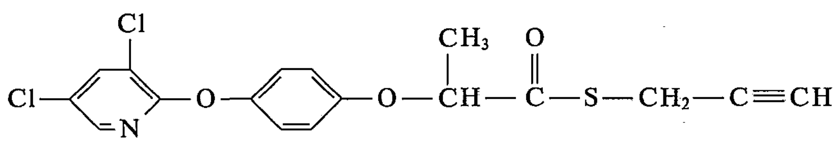

5. Die Verbindung der Formel

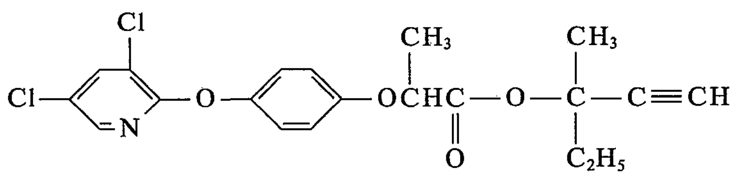

6. Die Verbindung der Formel

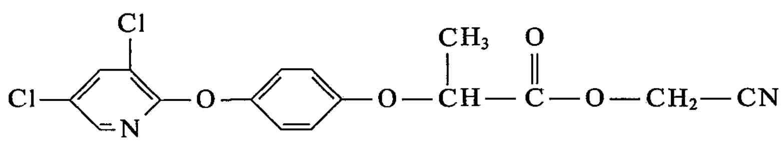

7. Die Verbindung der Formel

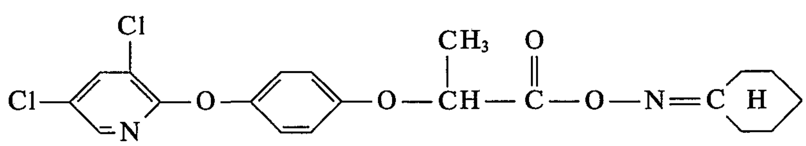

8. Verfahren zur Herstellung der neuen ungesättigten Ester der Formel I des Patentanspruchs 1, dadurch gekennzeichnet, daß man ein entsprechendes Propionsäurehalogenid der Formel II

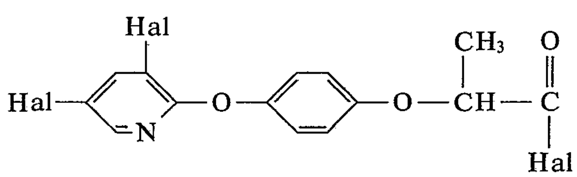

(II)

worin »Hal« je Chlor oder Brom bedeuten, in Gegenwart eines basischen Säureakzeptors mit einem ungesättigten Alkohol oder Thiol der Formel III

H—X—R (III)

worin R und X wie unter Formel I definiert sind, in an sich bekannter Weise umsetzt.

9. Verfahren zur Herstellung der neuen ungesättigten Ester der Formel I des Patentanspruchs 1, dadurch gekennzeichnet, daß man einen Hydroxyphenylpyridyläther oder ein Salz desselben, von der Formel IV

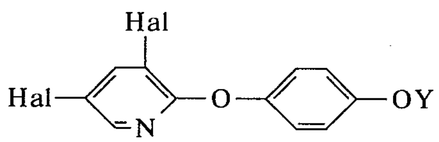

(IV)

worin die »Hal« je Chlor oder Brom bedeuten und Y Wasserstoff oder das Äquivalent eines Alkali- oder Erdalkalimetall-Kations darstellt, mit einem $\alpha$-Halogenpropion(thiol)säureester der Formel V

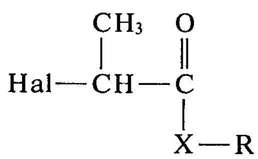

(V)

in Gegenwart eines säurebindenden Mittels umsetzt.

10. Herbizides Mittel, dadurch gekennzeichnet, daß es als wirksame Komponente einen ungesättigten 4-(3',5'-Dihalogen-pyridyl-2'-oxy)-$\alpha$-phenoxy-propion- oder -propionthiolsäure-Ester der Formel I des Patentanspruchs 1 bzw. der Unteransprüche 2 bis 7 enthält.

11. Die Verwendung der ungesättigten Ester der Formel I des Patentanspruchs 1 zur Bekämpfung von monocotylen Unkräutern.

12. Die Verwendung der ungesättigten Ester der Formel I zur selektiven post-emergenten Bekämpfung von monocotylen Unkräutern, wie Avena fatua, und Hirsearten in Kulturpflanzenbeständen, insbesondere in Getreide, Reis und Soja.

13. Verwendung eines der ungesättigten Ester der Ansprüche 3 bis 7 zur selektiven post-emergenten Bekämpfung von Avena fatua und Hirsearten in Weizen-, Gerste-, Reis- und Sojakulturen.

14. Verfahren zur selektiven Bekämpfung von monocotylen Ungräsern, wie Avena fatua und Hirsearten, in Kulturpflanzenbeständen wie Getreide, Reis, Soja etc., dadurch gekennzeichnet, daß man die verunkrauteten Kulturpflanzungen nach dem Auflaufen der Kulturpflanzen und Unkräuter mit einer wirksamen Menge eines Mittels gemäß Anspruch 10 behandelt, das als aktive Komponente einen ungesättigten Ester der Formel I des Patentanspruchs 1 enthält.

## Claims

1. A novel herbicidally active unsaturated ester of 4-(3',5'-dihalopyridyl-2'-oxy)-$\alpha$-phenoxypropionic acid or -thiopropionic acid of the formula

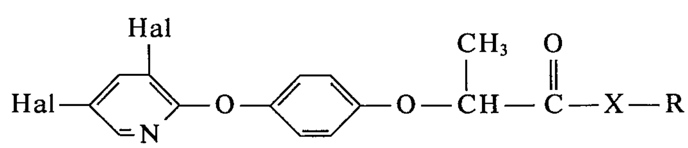

(I)

wherein each Hal independently is chlorine or bromine, X is an oxygen or a sulfur atom, R is a straight chain or branched $C_3-C_9$ alkynyl radical which is unsubstituted or substituted by chlorine and which, in addition to containing the tripe bond, can also contain a double bond, or is the 2-ethynylcyclohexyl-(1) radical, a straight chain or branched cyanoalkyl radical containing 1 to 5 carbon atoms in the alkyl moiety, the 1-cyanocyclohexyl-(1) radical or a radical of the formula

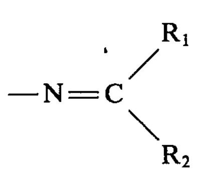

or

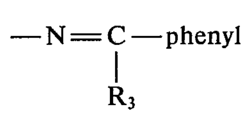

which is bonded to an oxygen atom X, in which formulae $R_1$ is hydrogen or an alkyl radical of 1 to 4 carbon atoms and $R_2$ is an alkyl radical of 1 to 4 carbon atoms, or $R_1$ and $R_2$ together with the adjacent

carbon atom form a cyclopropane or cyclohexane ring, and $R_3$ is hydrogen, methyl or a cyano group.

2. An unsaturated ester according to claim 1, wherein each Hal in formula I is chlorine.

3. The compound of the formula

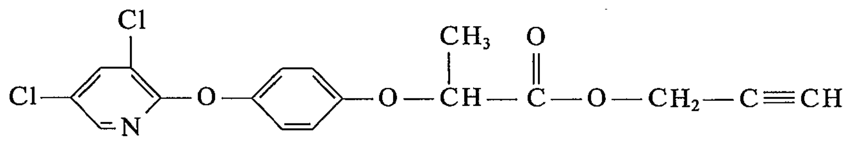

4. The compound of the formula

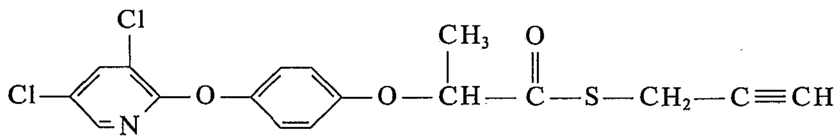

5. The compound of the formula

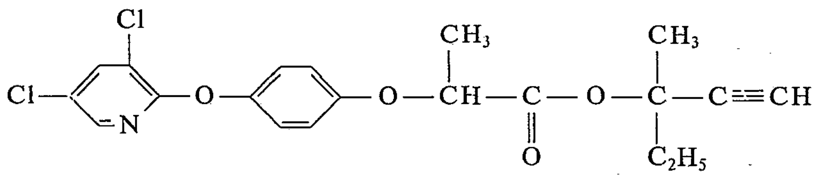

6. The compound of the formula

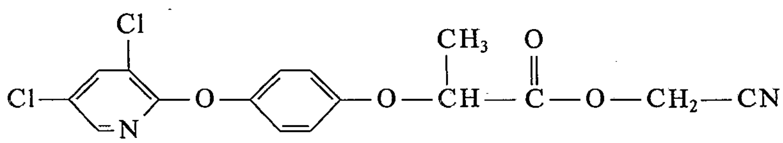

7. The compound of the formula

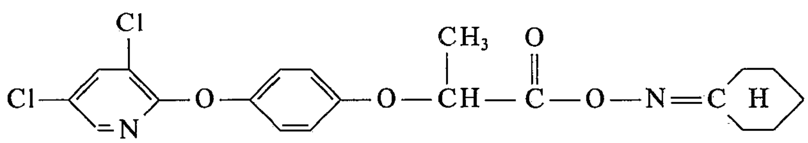

8. A process for the production of a novel unsaturated ester of the formula I according to claim 1, which process comprises reacting a corresponding propionic acid halide of the formula II

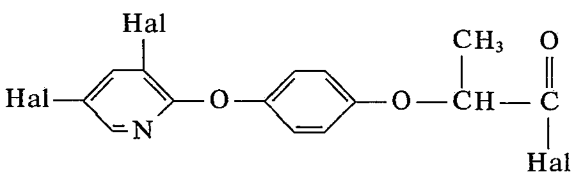

(II)

wherein each »Hal« is chlorine or bromine, with an unsaturated alcohol or thiol of the formula III

$$H—X—R \qquad (III)$$

wherein R and X are as defined for formula I, in a manner known per se, in the presence of a basic acid acceptor.

9. A process for the production of a novel unsaturated ester fo the formula I according to claim 1, which process comprises reacting a hydroxyphenyl pyridyl ether or a salt thereof, of the formula IV

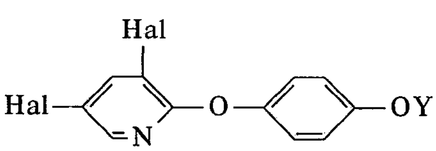

(IV)

wherein each »Hal« is chlorine or bromine and Y is hydrogen or the equivalent of an alkali metal cation or alkaline earth metal cation, with an $\alpha$-halopropionic or $\alpha$-halothiopropionic acid ester of the formula V

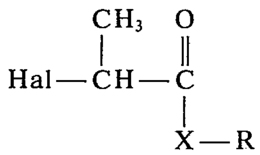

(V)

in the presence of an acid acceptor.

10. A herbicidal composition containing, as active ingredient, an unsaturated $\alpha$-(4-(3',5'-dihalopyridyl-2'-oxy)-phenoxy)-propionic or -thiopropionic acid ester of the formula I according to claim 1 or according to any one of subclaims 2 to 7.

11. Use of an unsaturated ester of the formula I according to claim 1 for controlling monocot weeds.

12. Use of an unsaturated ester of the formula I for the selective post-emergence control of monocot weeds, for example Avena fatua and species of millet, in crops of cultivated plants, especially in cereals, rice and soybeans.

13. Use of one of the unsaturated esters according to any one of claims 3 to 7 for the selective post-emergence control of Avena fatua and species of millet in crops of wheat, barley, rice and soybeans.

14. A method of selectively controlling monocot grasses, for example Avena fatua and species of millet, in crops of cultivated plants such as cereals, rice, soybeans and the like, which method comprises treating the weedinfested crops post-emergence with an effective amount of a composition according to claim 10, containing, as active ingredient, an unsaturated ester of the formula I according to claim 1.

**Revèndications**

1. Nouveaux esters insaturés d'acides 4-(3',5'-dihalogènpyridil-(2')-oxy)-$\alpha$-phénoxy-propioniques et -propionthioliques actifs comme herbicides, de formule

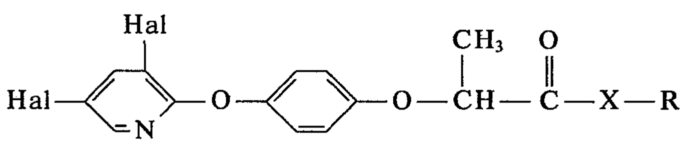

(I)

où Hal représente indépendamment l'un de l'autre un chlore ou un brome, X un atome d'oxygène ou de soufre, et R un radical alcynyle en $C_3$ à $C_9$ à chaîne droite ou ramifiée éventuellement substitué par un chlore, qui outre la triple liaison peut encore contenir une double liaison, le radical 2-éthynyl-cyclohexyl-(1), un radical cyanoalcoyle à chaîne droite ou ramifiée avec de 1 à 5 atomes de carbone dans la fraction alcoyle, le radical 1-cyano-cyclohexyl-(1) ou un radical lié avec un atome d'oxygène X de formule

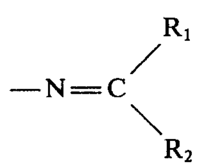

ou

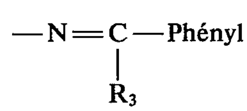

où $R_1$ représente un hydrogène ou un radical alcoyle en $C_1$ à $C_4$ et $R_2$ un radical alcoyle en $C_1$ à $C_4$, où $R_1$ et $R_2$ forment ensemble avec l'atome de carbone voisin un noyau cyclopentane ou cyclohexane et $R_3$ représente un hydrogène, un méthyle ou un groupe cyano.

2. Esters insaturés selon la revendication 1, caractérisé en ce que »Hal« dans la formule I représente à chaque fois un chlore.

3. Composé de formule

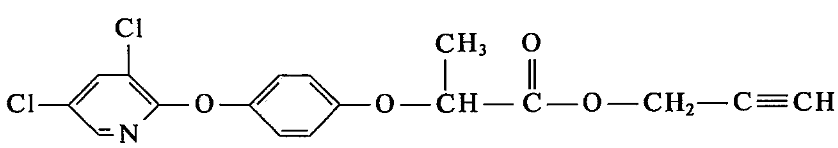

4. Composé de formule

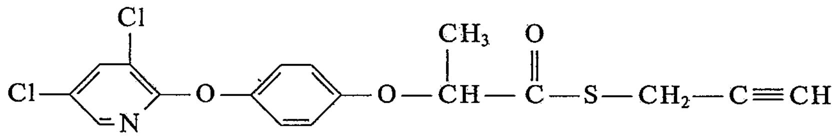

5. Composé de formule

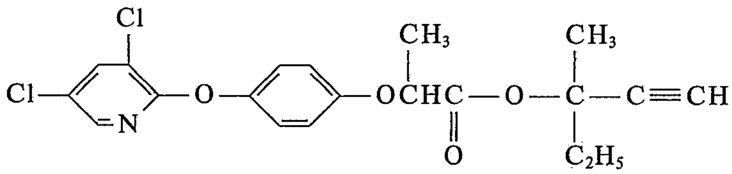

6. Composé de formule

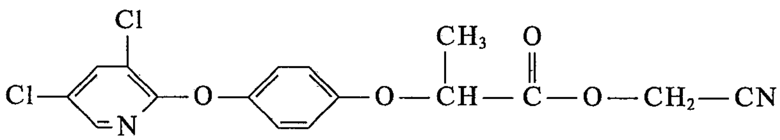

7. Composé de formule

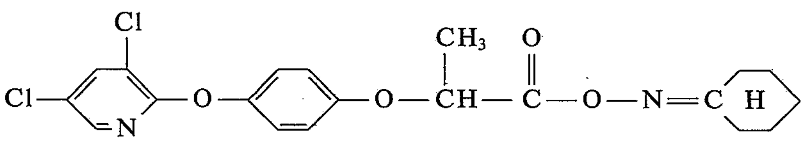

8. Procédé de préparation des nouveaux esters insaturés de formule I de la revendication 1, caractérisé en ce qu'on fait réagir un halogènure d'acide propionique correspondant de formule II

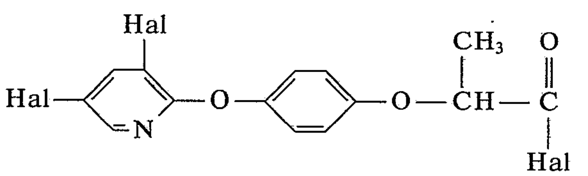 (II)

où les »Hal« représentent chacun un chlore ou un brome, en présence d'un accepteur d'acide basique avec un alcool ou un thiol insaturé de formule III

$$H—X—R \qquad (III)$$

où R et S sont tels que définis pour la formule I, et ce de façon classique.

9. Procédé de préparation des nouveaux esters insaturés de formule I de la revendication 1, caractérisé en ce qu'on fait réagir un hydroxyphényl-pyridyléther ou un de ses sels de formule IV

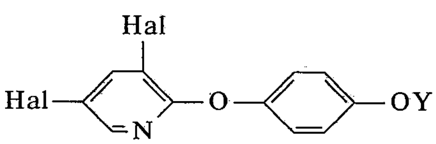 (IV)

où les »Hal« représentent chacun un chlore ou un brome et Y un hydrogène ou l'équivalent d'un cation de métal alcalin ou alcalino-terreux, avec un ester d'acide $\alpha$-halogènepropion(thil)ique de formule V

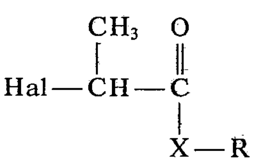 (V)

en présence d'un agent liant acide.

10. Agent herbicide, caractérisé en ce qu'il contienent comme composant actif un ester insaturé d'acide 4-(3',5'-Dihalogen-pyridyl-2'-oxy)-$\alpha$-phénoxy-propionique ou -propionthiolique de formule I de la revendication 1 ou des revendications 2 à 7.

11. Application des esters insaturés de formule I de la revendication 1 à la lutte contre les mauvaises herbes monocotylédones.

12. Application des esters insaturés de formule I à la lutte sélective en post-levée contre les mauvaises herbes monocotylédones comme Avena fatua et les différentes espèces de sorgho dans les plantes cultivées, en particulier les céréales, le riz et le soja.

13. Application de l'un des esters insaturés des revendications 3 à 7 à lutte sélective en post-levée contre Avena fatua et les différentes espèces de sorgho dans les cultures de blé, d'orge, de riz et de soja.

14. Procédé de lutte sélective contre les graminées indésirables monocotylédones comme Avena fatua et les différentes espèces de sorgho dans les cultures comme les céréales, le riz, le soja, etc, caractérisé en ce qu'on traite les cultures envahies par les mauvaises herbes après la levée des plantes cultivées et des mauvaises herbes avec une quantité efficace d'un agent selon la revendication 10 qui contient comme composant actif un ester insaturé de formule I de la revendication 1.